# EUROPEAN PATENT SPECIFICATION

(11) **EP 1 418 931 B1**
(45) Date of publication and mention of the grant of the patent: **25.06.2008**
(21) Application number: 02760977.5
(22) Date of filing: 26.08.2002
(51) Int. Cl.: A61K 38/00, C07K 14/705, G01N 33/566

(54) **NEW ANGIOGENESIS INHIBITORS BASED ON SOLUBLE CD44 RECEPTOR HYALURONIC ACID BINDING DOMAIN**
NEUE ANGIOGENESE-INHIBITOREN BASIEREND AUF LÖSLICHER HYALURONSÄURE-BINDEDOMÄNE DES CD44-REZEPTORS
NOUVEAUX INHIBITEURS DE L'ANGIOGENESE BASES SUR LE DOMAINE DE FIXATION DE L'ACIDE HYALURONIQUE DU RECEPTEUR CD44 SOLUBLE

(30) Priority: 24.08.2001 SE 0102823; 24.08.2001 US 314971 P
(43) Date of publication of application: 19.05.2004
(73) Proprietor: Celecure AS, 12618 Tallinn (EE)
(72) Inventor: Staffan Strömblad, 141 44 Huddinge (SE); Kogerman, Priit, 76901 Tabasalu (EE); Päll, Taavi, 12618 Tallin (EE)
(74) Representative: Larsson, Karin
(86) International application number: PCT/SE2002/001531
(87) International publication number: WO 2003/018044

(56) References cited:
- WO-A1-94/09811
- WO-A1-99/45942
- US-A- 5 863 540
- US-A- 5 916 561
- US-A- 6 010 865
- BAJORATH J. ET AL: 'Identification of CD44 residues important for hyaluronan binding and delineation of the binding site' THE JOURNAL OF BIOLOGICAL CHEMISTRY vol. 273, 1998, pages 338 - 343, XP002976065
- BAJORATH J.: 'Molecular organization, structural features and ligand binding characteristics of CD44, a highly variable cell surface glycoprotein with multiple functions' PROTEINS, STRUCTURE, FUNCTION AND GENETICS vol. 39, 2000, pages 103 - 111, XP001053566
- AHRENS T. ET AL: 'Soluble CD44 inhibits melanoma tumor growth by blocking cell surface CD44 binding to hyaluronic acid' ONCOGENE vol. 20, 2001, pages 3399 - 3408, XP002976066
- ALPAUGH M.L. ET AL: 'Myoepithelial-specific CD44 shedding contributes to the anti-invasive and antiangiogenic phenotype of myoepithelial cells' EXPERIMENTAL CELL RESEARCH vol. 261, 2000, pages 150 - 158, XP002976067

## Description

### Technical field

The invention refers to the use of a molecule comprising a non-HA-binding variant of the CD44-hyaluronic acid binding domain for the manufacturing of a medicament. Furthermore, the invention relates to a method for screening for substances binding to the molecule comprising a non-HA-binding variant of the CD44-hyaluronic acid binding domain.

### Technical background

The formation of new blood vessels by angiogenesis is a central event in many different pathological states, including ocular diseases causing blindness, such as macular degeneration, diabetic retinopathy and states of retinal hypoxia, states of chronical inflammation, such as rheumatoid arthritis, in psoriasis, atherosclerosis, restenosis, as well as in cancer growth and metastasis. In addition, hemangioma is caused by uncontrolled proliferation of endothelial cells. Given that many of these diseases are of a chronical nature and presently lack satisfactory medicaments, makes the search for treatments and drugs against these diseases very important. To this end, an agent blocking angiogenesis has the potential to constitute a medicament for all these common angiogenesis- (and/or endothelial cell-)dependent diseases.

One interesting target for drugs against diseases of this kind has been CD44 (Naot et al., Adv Cancer Res 1997;71:241-319). CD44 is a cell surface receptor for the large glycosaminoglycan of the extracellular matrix hyaluronic acid (HA) [Aruffo et al., Cell 1990; 61:1303-13]. CD44 plays a role in various cellular and physiological functions, including adhesion to and migration on HA, HA degradation and tumour metastasis. The CD44 receptor shows a complex pattern of alternative splicing in its variable region of the extracellular domain [Screaton et al., PNAS 1992; 89: 12160-4]. CD44 is able to bind matrix metalloproteinase-9 (MMP-9) and can thereby localize MMP-9 to the cellular membrane, which may in part explain its activity in promoting tumour cell invasion and metastasis [Yu, 1999 #3].

Among patent references disclosing CD44 and its connection to diseases described above may US-A-6025138, US-A-5902795, US-A-6150162, US-A-6001356, US-A-5990299 and US-A-5951982 be mentioned.

WO94/09811 describes the use of CD44 in treating inflammation or detecting cancer metastasis of hematopoietic origin. Use of CD44 for inhibiting solid tumor growth or angiogenesis is not disclosed. WO 99/45942 discloses the use of HA-binding proteins and peptides including CD44 to inhibit cancer and angiogenesis-dependent diseases. CD44 is mentioned as one example of a long list of HA-binding proteins. In both publications the use of CD44 is limited to its ability to bind hyaluronic acid.

Ahrens et al. (Oncogene 2001; 20; 3399-3408) discloses that soluble CD44 inhibits melanoma tumour growth by blocking the binding of tumour cell surface CD44 to hyaluronic acid. Thus, this work teaches a hyaluronic acid binding dependent mechanism for the CD44 effect directly on melanoma tumour cell growth.

Alpaugh et al. (Exp. Cell Res. 261, 150-158 (2000)) discloses myoepithelial-specific CD44 and its antiangiogenic properties. This study deals with HA-binding properties of CD44.

Bajorath (PROTEINS: Structure, Function, and Genetics 39: 103-111 (2000)) discloses CD44 and its binding to HA, cell adhesion and CD44-signalling. Moreover, CD44 mutagenesis experiments are disclosed involving among others the well-established non-HA-binding mutations R41A and R78S, and their impact on CD44-binding to HA.

However, in all these cases, the non-HA-binding CD44-mutants have been used as negative controls for HA-binding properties and no biological activity has been attributed to the non-HA-binding mutants.

Thus, the prior art discloses the potential use of CD44 to specify that any effects are dependent on HA-CD44-interaction. Consequently, all utility ascribed this far to CD44-derived peptides is directly dependent on their ability to bind hyaluronic acid.

Given that hyaluronic acid is widely expressed in the body at high levels, a treatment based on inhibition of this extracellular component result in a high risk for unwanted side effects outside of the tumour. Furthermore, because of the high total amounts of HA in the body, such strategy will require high doses of HA-blocking recombinant proteins, even increasing the risk for side effects.

Accordingly, a need exists for finding novel drugs for treating tumours, as well as novel pathways for the relation between CD44 and tumour growth, in order to provide new drug targets, which avoid the side-effects described above.

In addition, there is a need to develop novel inhibitors of angiogenesis, as these constitute potential medicaments not only for cancer, but also for an array of common diseases as disclosed above. To this end, it is important to elucidate the relation between CD44 and angiogenesis, in particular the potential direct effects of CD44 on the vasculature and on the various diseases that are dependent on new blood vessel formation.

### Summary of the invention

Kogerman et al. [Kogerman et al., Oncogene 1997; 15: 1407-16] found that mouse fibrosarcoma cells stably expressing human CD44 standard isoform (hCD44s) had lost their hCD44s expression in large subcutaneous tumours. When hCD44s negative cells from these primary tumours were reintroduced subcutaneously into new mice for second round of tumour growth, then resulting tumours had significantly shorter latency times than hCD44s positive tumours.

The observed longer latency times for hCD44s expressing tumours lead the inventors to realize that the inhibitory effect of hCD44s overexpression in subcutaneous tumour growth is connected to inhibition of tumour angiogenesis. Induction of angiogenesis is essential for growth and persistence of solid tumours and their metastases. In the absence of angiogenesis, tumours cannot grow beyond a minimal size and remain dormant in the form of micrometastases [Holmgren et al., Nat Med 1995; 1: 149-53]. The inventors have discovered here that recombinant soluble human CD44 hyaluronic acid binding (CD44HABD) domain inhibits angiogenesis in vivo in chick and endothelial cell proliferation in vitro, and thereby blocks human tumour growth in chick and mice. The inventors describe a novel type of angiogenesis inhibitor, as they found that recombinant cell surface receptor CD44 inhibits angiogenesis and tumour growth in vivo and endothelial cell proliferation in vitro. The inventors have created mutant forms of CD44 that are capable of inhibiting angiogenesis. The advantage with these mutants of CD44 is that they do not bind HA, demonstrating that the mechanism for inhibition of angiogenesis is independent of binding to HA. Importantly, use of mutant CD44 for systemic administration as a medicament will be more specific for angiogenesis, since it will not be bound up by HA in the body, and can therefore be used at lower doses and has less risk of causing unwanted side effects.

Accordingly, the invention refers to the use of a molecule comprising a non-HA-binding variant of the CD44-hyaluronic acid binding domain, as well as analogues and recombinant variants thereof, including the specified mutants, for the manufacturing of a medicament for treating states related to the inhibition of angiogenesis. Moreover, the invention refers to a method for screening for molecules binding to a non-HA-binding variant of the CD44-hyaluronic acid binding domain, thereby being potential targets for inhibiting angiogenesis and for cell proliferation.

Also, the invention refers to a molecule comprising a non-HA-binding variant of the CD44-hyaluronic acid binding domain, as well as analogues, recombinant and mutated variants thereof for targeting of endothelial cells. Hereby, drugs for treating states related to angiogenesis, such as various cancerous states, are easily provided by the invention, taking advantage of the novel mechanisms presented by the inventors. Furthermore, through the method of screening, other molecules may be found, which affect cell proliferation and/or angiogenesis.

### Short description of the drawings

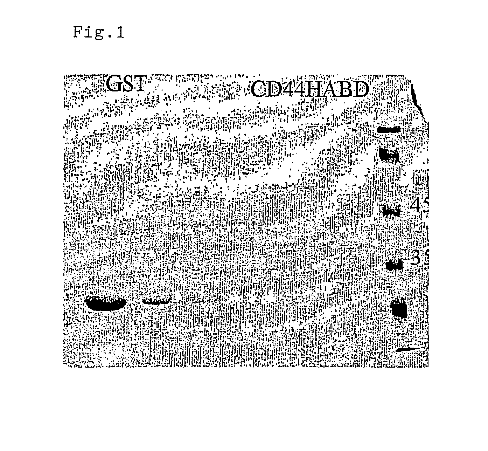

Figure 1 shows a SDS PAGE of recombinant GST and GST-CD44 proteins. The gel was stained with Coomassie Brilliant Blue. Molecular weight markers are shown on the right.

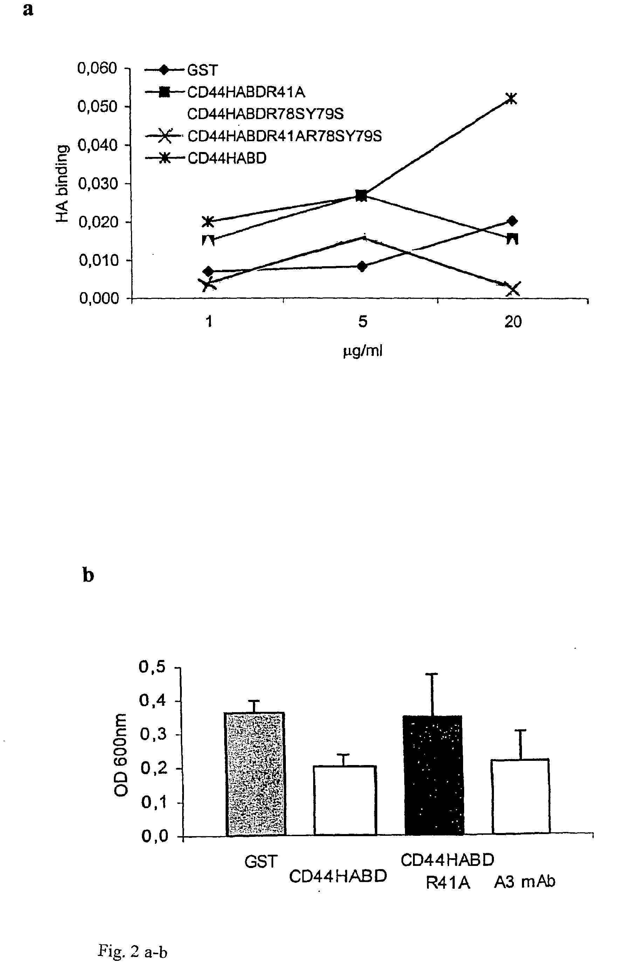

Figure 2 shows that recombinant CD44 hyaluronic acid binding domain binds to hyaluronic acid. **a**, wild type CD44HABD, but not R41A, R78SY79S or R41AR78SY79S mutants, binds to immobilized HA. **b**, CD44HABD inhibits human aortic endothelial cell migration towards HA, whereas R41A HA-non-binding mutant has no effect. A3, monoclonal antibody to CD44, that blocks HA binding, and also inhibit endothelial cell migration.

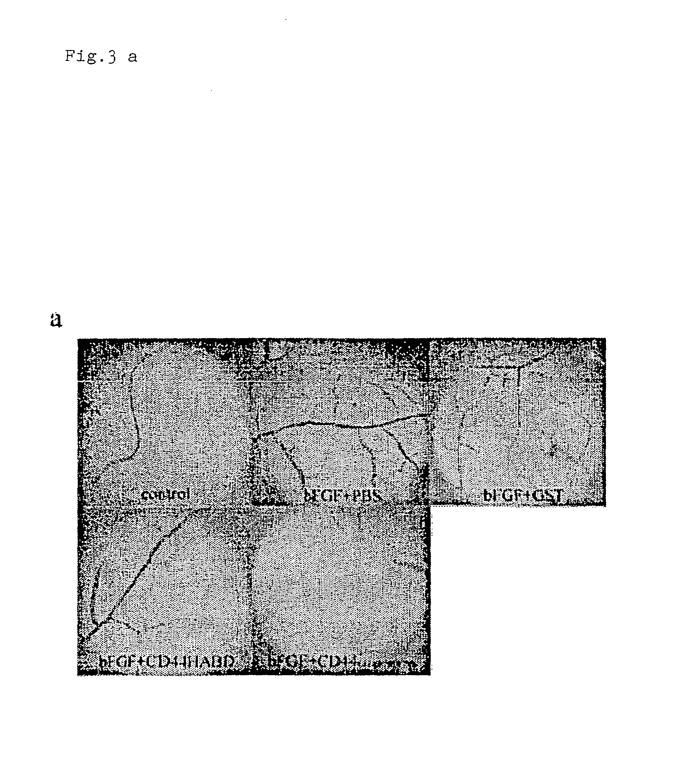

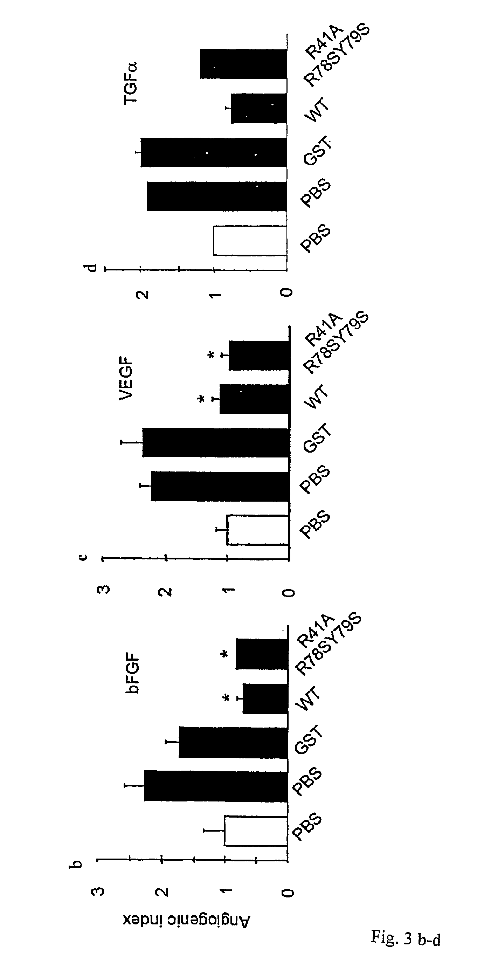

Figure 3 shows that recombinant human CD44 hyaluronic acid binding domain blocks angiogenesis in vivo in chick chorio-allantoic membrane. **a**, Filter discs and associated CAM from typical angiogenesis experiment where angiogenesis was induced with bFGF. **b-d**, Angiogenesis was assessed as number of blood vessel branch points; mean angiogenic index ± SEM. *(P<0.05), significant results.

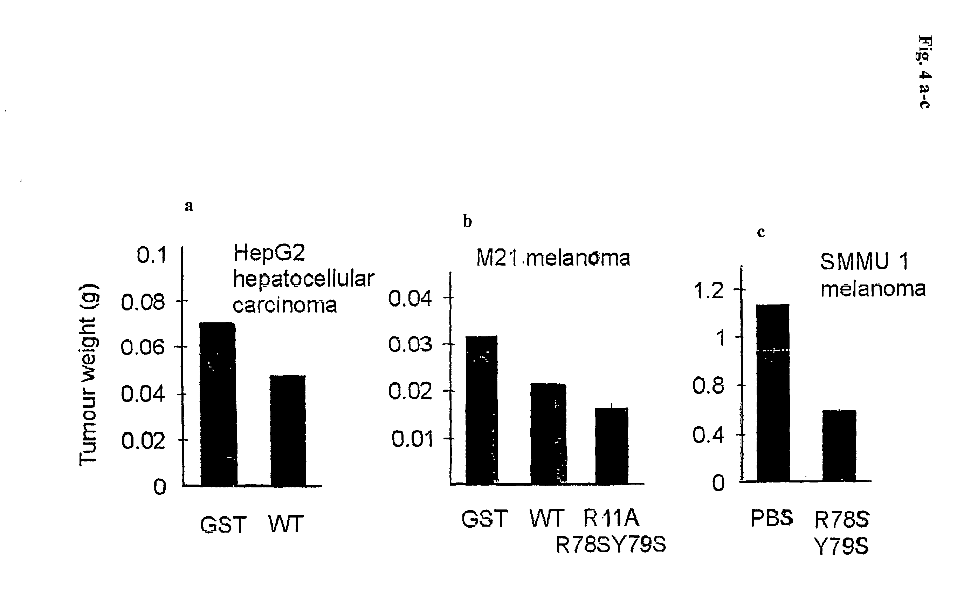

Figure 4 shows that recombinant CD44 hyaluronic acid binding domain inhibits melanoma (SMMU-1, M21) and hepatocellular carcinoma (HepG2) growth in chick CAM.

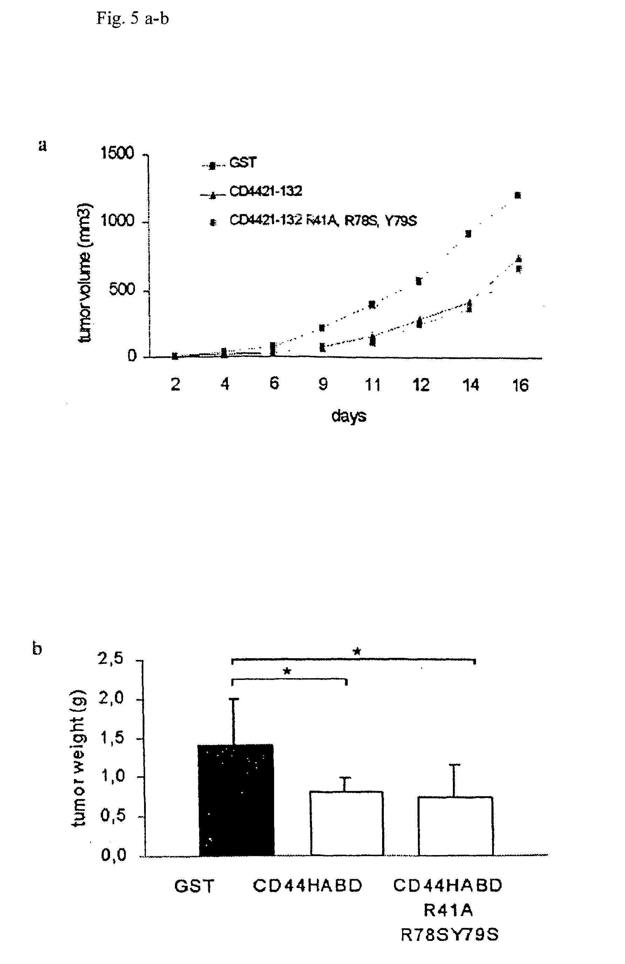

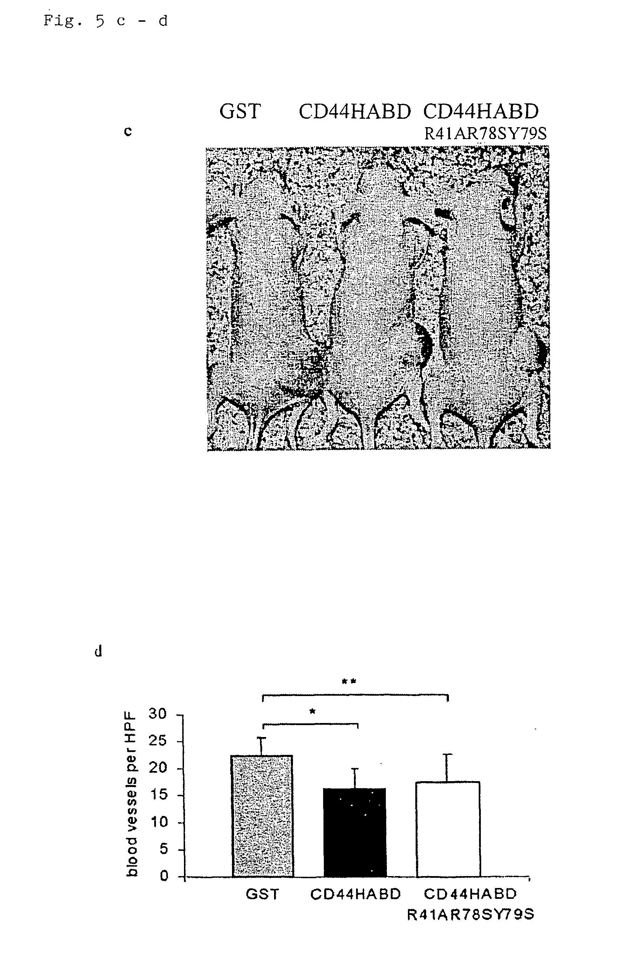

Figure 5 shows that recombinant CD44 hyaluronic acid binding domain inhibits CD44 negative melanoma growth. **a**, Growth curve of s.c. SMMU-1 tumours in nude mice treated with CD44HABD, CD44HABD_{R41AR78SY79S} or GST as control, n = 8 per group. **b**, Tumour weights at day 16, where the black line represents the median value. **c**, Representative photographs of mice at day 16, treated as indicated. **d**, Blood vessel density at tumour border per high power field (HPF). *(P<0.005), **(0.05) significant results.

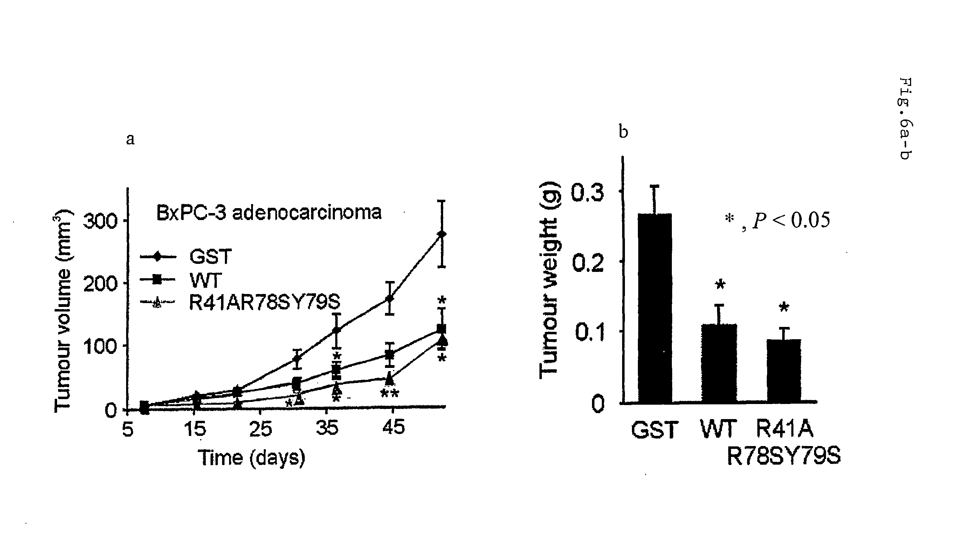

Figure 6. CD44-HABD fusion proteins inhibit the growth of human pancreatic cancer cells in nude mice. BxPC-3 pancreatic adenocarcinoma (***a***) tumours in nude mice treated with GST-CD44HABD (■), GST-CD44HABD^{R41AR78SY79S}(▲) or GST (◆) as control (*n* = 6-7). ***b**,* average BxPC-3 tumour weights at the end of experiment. Values in graphs and bars represent mean ± s.e.m. Asterisk indicates *P* < 0.05.

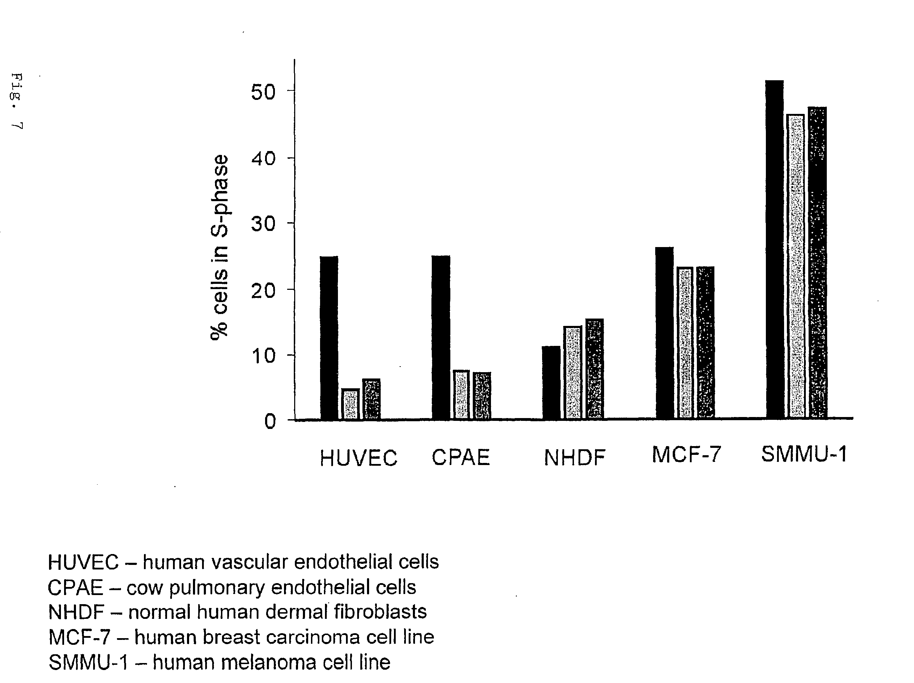

Figure 7. Recombinant CD44HABD blocks specifically endothelial cell cycle. The proportion of cells in S-phase when treated with GST (■), GST-CD44HABD (□) or GST-CD44HABD^{R41AR78SY79S} (□).HUVEC, human vascular endothelial cells; CPAE, cow pulmonary arteria endothelial cells; NHDF, normal human dermal fibroblasts; MCF-7, human breast carcinoma cells.

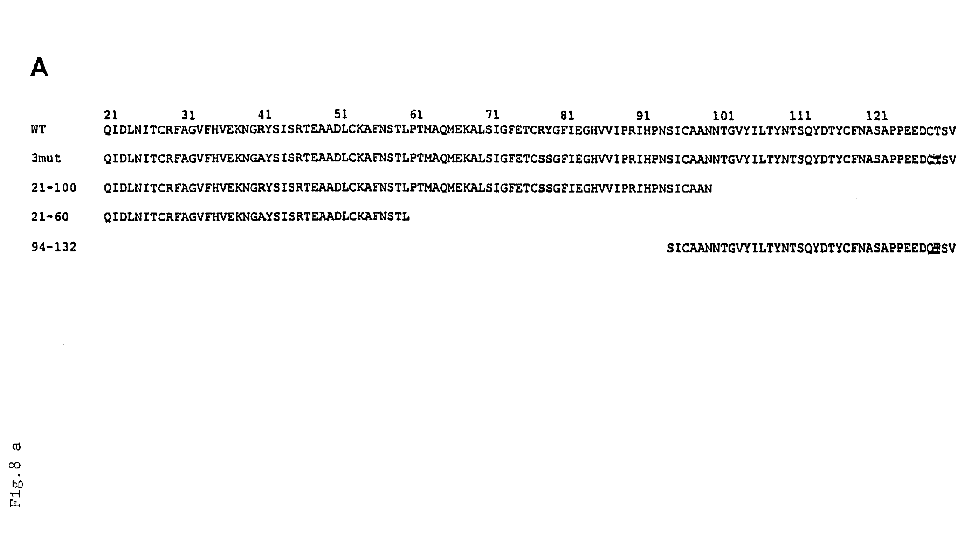

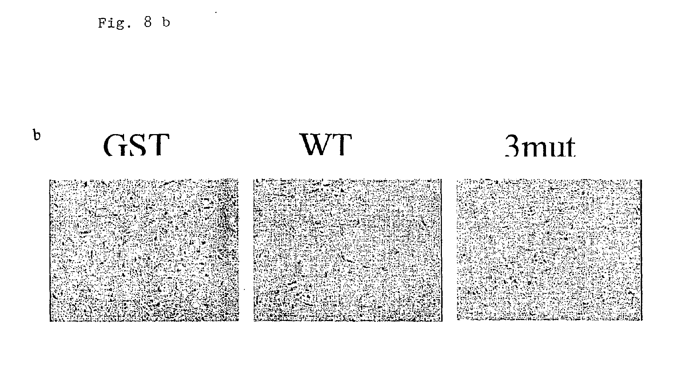

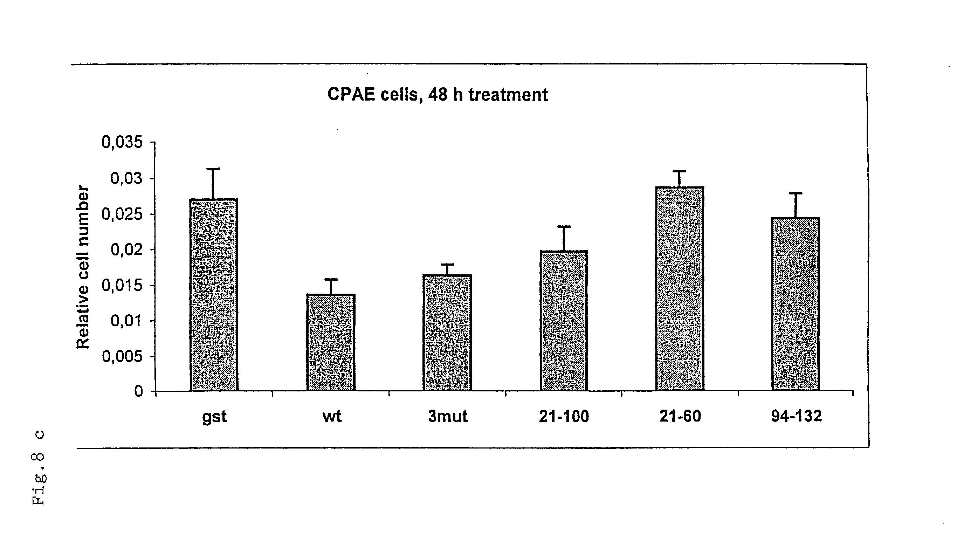

Figure 8 shows the effect of CD44HABD deletion mutants on endothelial cell proliferation. CD44HABD deletion mutants were derived from 3mut CD44HABD (mutated positions are indicated in bold, Fig 8A), recombinant protein was produced and purified as described in Example 1. Cow pulmonary endothelial cells (CPAE) were treated 48 h with indicated proteins (final conc. 15 µg/ml and 30 µg/ml, respectively) in medium containing 10% serum. Cell proliferation was assayed by photomicrography (B) or by MTT staining (Sigma) and spectrophotometry (C).

### Definitions

The "hyaluronic acid binding domain" is hereafter referred to as HABD

By a "non-HA-binding variant" of HABD is meant a variant that is modified by way of mutation or in any other way, so that it at least partly has lost its ability to bind to HA, but still has the capacity to inhibit angiogenesis and/or endothelial cell proliferation.

By "analogues and recombinant variants" of a molecule comprising the CD44-HABD are meant molecules, such as fusion proteins, comprising the CD44-HABD, thereby at least partly exerting essentially the properties of the CD44-HABD.

By "states related to the inhibition of angiogenesis and/or endothelial cell proliferation" are meant such states and diseases, which may be treated or affected by an inhibition of the angiogenesis and/or endothelial cell proliferation.

By "a binding partner" for a molecule comprising the CD44-HABD is meant a molecule having affinity for CD44-HABD or mutants thereof.

By "a receptor molecule, or a part of a receptor molecule" is meant a molecule acting as a receptor, or being part of a receptor.

By "a modified variant" is in the context of the invention meant any modification to a normal wt-molecule, such as deletions, insertions, substitutions, analogs, fragments or recombinant variants thereof.

### Detailed description of the invention

WO94/09811 describes the use of CD44 in treating inflammation or detecting cancer metastasis. The authors show that CD44 is upregulated in inflammatory conditions and CD44 peptides are capable of inhibiting T-cell activation. No data or claims are presented on inhibition of metastasis by CD44 and no claims are made towards use of CD44 for inhibiting tumor growth or angiogenesis. WO 99/45942 discloses the use of HA-binding proteins and peptides including CD44 to inhibit cancer and angiogenesis-dependent diseases. This publication uses metastatin, a 38 kDa fragment of the cartilage link protein as well as a HA-binding peptide derived from this fragment to inhibit pulmonary metastasis of B16 mouse melanoma and Lewis lung carcinoma. In the case of the HA-binding peptide, growth of B16 melanoma on chicken CAM and endothelial cell migration on HA have been inhibited.
In both publications the use of HA-binding peptides is directly related to their ability to bind hyaluronic acid.

CD44 was previously implicated to promote angiogenesis by a mechanism dependent on its ability to bind matrix metalloproteinase-9 (MMP-9) (Yu and Stamenkovic, Genes Dev 1999; 13: 35-48; Yu and Stamenkovic, Genes Dev 2000; 14: 163-76). Overxpression of soluble CD44 (sCD44v6-10) in murine TA3 mammary carcinoma cells inhibited the binding of MMP-9 to the tumour cell surface and thereby blocked tumour growth and vascularisation (Yu and Stamenkovic 2000). MMP-9 was previously demonstrated to be involved in angiogenesis during development and in tumours (Vu et al., Cell 1998; 93: 411-22; Bergers et al., Nat Cell Biol 2000; 2: 737-44; Coussens et al., Cell 2000; 103: 481-90). CD44-NFVIP-9 complex is also implicated in activation of latent TGFβsince tubulogenesis in vitro was inhibited by block of TGFβ(Yu and Stamenkovic, 2000)̃.

The mutants of CD44-HABD used by the inventors show very different affinities towards MMP-9 but independent of that inhibit angiogenesis equally well, thereby making it unlikely that MMP-9 binding is critical for the inhibition of angiogenesis disclosed in the present invention. In addition, the inventors describe a mechanism for CD44 that directly inhibits angiogenesis. Furthermore, the inventors demonstrate a mechanism for CD44 that has a distinct target in normal endothelial cells, compared to the previously proposed mechanism disrupting CD44-binding of MMP-9 at transformed tumour cell surfaces. Gao et al. (Cancer Res 1998; 58: 2350-2) show that metastatic ability but not tumourigenicity of rat Dunning AT3.1 prostate cancer cells is independent of HA binding, as overexpression of rat CD44 standard isoform and R44A non-HA-binding mutant both reduced dramatically formation of metastatic lung colonies but not local tumour growth. This suggests that other CD44 binding partners distinct from HA must be involved in metastasis. A number of CD44-binding proteins have been described including HGF, bFGF, fibronectin, osteopontin, selectin to name a few. However, the binding of several of these proteins is dependent on the post-translational modifications of CD44 and/or the inclusion of alternative exons in CD44 that are not present in our recombinant fusion proteins. Also, many of these CD44-binding proteins are present in large amount in the body, making CD44-derivatives binding to any of these less useful as medicament, because of a high risk of side effects. In addition, none of the previously described proteins are unique for targeting vascular cells, neither do they block a pathway required specifically for the growth of endothelial cells. Therefore, both this ligand and this pathway must be novel. The invention discloses a method for identifying the binding partner of CD44 and the pathway that is relevant for the inhibition of angiogenesis and endothelial cell growth.

In a first aspect the invention relates to the use of a molecule comprising a non-HA-binding variant of the CD44-hyaluronic acid binding domain, as well as analogues, recombinant and mutated variants thereof, for the manufacturing of a medicament for treating states related to the inhibition of angiogenesis and/or endothelial cell proliferation.

In one embodiment, the CD44-HABD comprises at least one mutation, thereby rendering it non-HA-binding.

In a preferred embodiment, the mutation(s) is (are) chosen from F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A, F119Y. Preferably, the mutations are chosen from one or more of R41A, R78S, Y79S. Also, deletion mutations resulting in any fragment of CD44 from 3 to 110 amino acids in length are potentially useful for the purposes of the invention. However, the skilled person easily realises that any mutation to wild-type HA-binding CD44, which makes the CD44-HABD, or fragments thereof, at least partly non-HA-binding, such as one or more deletions, substitutions or additions, may be introduced in the CD44-HABD part, as long as the desired properties are achieved.

The CD44-HABD is a protein covering amino acids 21-132 of intact human CD44 molecule, or has high degree of homology to this region of human CD44. The chicken CD44-HABD is the most dissimilar HABD that has been isolated by the inventors, having a sequence homology of 55% to human HABD at the amino acid level. Thus, a high degree of sequence homology means at least approximately 55% amino acid homology, desirably at least 65 % homology, and most desirably at least 75% homology.

Furthermore, the molecule according to the invention refers to a deleted or in any other way changed or mutated form of the CD44-HABD protein, whereby the changed form exhibits essentially the same properties as the original CD44-HABD-protein, or the herein specified CD44-HABD-mutants, as measured by any one of the methods described here.

In a preferred embodiment, the molecule comprising the non-HA-binding variant of the CD44-hyaluronic acid binding domain is chosen from the group comprising: human CD44-HABD (SEQ ID NO:2), dog CD44-HABD (SEQ ID NO:4), chick CD44-HABD (SEQ ID NO:6), human CD44-HABD-R41A (SEQ ID NO:8), human CD44-HABD-R78SY79S (SEQ ID NO:10), and CD44-HABD-R41AR78SY79S (SEQ ID NO:12), the sequences above further comprising at least one modification thereby making them non-HA-binding. Other variants are also possible, such as CD44-HABD-R78S, CD44-HABD-Y79S, as well as GST-CD44-HABD-fusion proteins having the R41A, R78S or the Y79S mutations.

CD44-HABD-R41A, CD44-HABD-R78SY79S and CD44-HABD-R41AR78SY79S are preferred examples of mutated variants of CD44-HABD, wherein the last four letters/figures indicates the position and type of mutation.

GST-CD44-HABD is a fusion protein of a GST-part and CD44-HABD. Other possible fusion proteins may be chosen from the group comprising IgG, IgM, IgA, His, HA, FLAG, c-myc, EGFP. GST is a short for glutathione-S-transferase, being used as a tag for the purpose of being able to purify the fusion protein on a GST-binding column, as well as for the purpose of detection. GST occurs naturally as a 26 kDa protein (Parker, M.W. et al., J. Mol. Biol. 213, 221 (1990); Ji, X. et al., Biochemistry 31, 10169 (1992); Maru, Y. et al., J. Biol. Chem. 271, 15353 (1996).).

Accordingly, in another embodiment, the recombinant variant is a fusion protein having a GST part and a CD44-HABD part, wherein the CD44-HABD-part is in a wild-type form or in a mutated form. Other tags than GST are also fully possible.

Preferably, the CD44-hyaluronic acid binding domain has a homology to the sequence SEQ ID NO:2 of at least 55 %, more preferably at least 65 %, even more preferably at least 75%. Most preferably, the CD44-hyaluronic acid binding domain is a modified variant (non-HA-binding gene product) of the sequence SEQ ID NO:1.

The states and diseases to be treated may be any one chosen from the following group: ocular diseases causing blindness, or impaired vision, such as macular degeneration, diabetic retinopathy and states of retinal hypoxia, states of chronical inflammation, such as rheumatoid arthritis, in psoriasis, atherosclerosis, restenosis, as well as in cancer growth and metastasis, as well as all forms of cancer diseases and tumours, such as a cancer of breast, prostate, colon, lung, skin, liver, brain, ovary, testis, skeleton, epithelium, endothelium, pancreas, kidney, muscle, adrenal gland, intestines, endocrine glands, oral cavities, head and neck, or other solid tissue origin, or being any form of leukemia, as well as in hemangioma.

The invention may be used in human and veterinary medicine. For instance, the invention may be used for mouse, rat, chick, dog, horse, cat, bovine animals and for all long-lived species in a normal zoo. Preferably, the invention is used for humans.

In still another aspect, the invention refers to a recombinant molecule comprising a GST-part and a CD44-HABD part. The CD44-HABD part may for example be mutated with one or more of the following mutations: F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A, F119Y. Preferably, the mutations are chosen from one or more of R41A, R78S, Y79S. However, the skilled person easily realises that any mutation to wild-type HA-binding CD44, which makes the CD44-HABD, or fragment thereof, at least partly non-HA-binding, such as one or more deletions, substitutions or additions, may be introduced in the CD44-HABD part, as long as the desired properties are achieved.

In a preferred embodiment, the CD44-HABD-part comprises a non-HA-binding variant of the CD44-HABD of any one of the amino acid sequences SEQ ID NO:2, SEQ ID NO:4, SEQ ID NO:6, SEQ ID NO:8, SEQ ID NO:10, SEQ ID NO:12, SEQ ID NO: 23, SEQ ID NO:26, the sequences above further comprising at least one modification thereby making them non-HA-binding.

In the most preferred embodiment, CD44-HABD comprises at least three consecutive amino acids of the amino acids 23-132 of CD44. Basically, all fragments from 3-110 amino acids in size are potentially efficient, for example amino acids 23-25, 24-26, 25-27, etc., amino acids 23-26, 24-27, etc., 23-27, etc., 23-28, etc. Thus, all combinations of consecutive amino acids up to 110 amino acids of the original molecule are possible for the purposes of the invention, as the skilled man easily realises,a s long as they show the desired properties, as tested by the methods illustrated in the example section of this application

In another embodiment, the CD44-HABD part is encoded by a sequence having at least 55% homology, preferably 65% homology, more preferably 75% homology, most preferably being any one of the nucleotide sequences: SEQ ID NO:1, SEQ ID NO:3, SEQ ID NO:5, SEQ ID NO:7, SEQ ID NO:9, SEQ ID NO:11, whereby the nucleotide sequences are in a modified form (non-HA-binding gene product or peptide).

In another aspect, the invention refers to a pharmaceutical composition in order to inhibit angiogenesis and/or endothelial cell proliferation, characterised in that it comprises at least one molecule comprising the CD44-hyaluronic acid binding domain, as well as analogues, recombinant and mutated variants thereof, in mixture or otherwise together with at least one pharmaceutically acceptable carrier or excipient.

The pharmaceutical compositions are prepared in a manner known to a person skilled in the pharmaceutical art. The carrier or the excipient could be a solid, semisolid or liquid material that could serve as a vehicle or medium for the active ingredient. Suitable carriers or excipients are known in the art. The pharmaceutical composition could be adapted to oral or parenteral use and could be administered to the patient as tablets, capsules, suppositories, solutions, suspensions or the like.

The pharmaceutical compositions could be administered orally, e.g. with an inert diluent or with an edible carrier. They could be enclosed in gelatin capsules or be compressed to tablets. For oral therapeutic administration the compounds according to the invention could be incorporated with excipients and used as tablets, lozenges, capsules, elixirs, suspensions, syrups, wafers, chewing gums and the like. These preparations should contain at least 4% by weight of the compounds according to the invention, the active ingredient, but could be varied according to the special form and could, suitably, be 4-70% by weight of the unit. The amount of the active ingredient that is contained in compositions is so high that a unit dosage form suitable for administration is obtained.

The tablets, pills, capsules, lozenges and the like could also contain at least one of the following adjuvants: binders such as microcrystalline cellulose, gum tragacanth or gelatin, excipients such as starch or lactose, disintegrating agents such as alginic acid, Primogel, corn starch, and the like, lubricants such as magnesium stearate or Sterotex, glidants such as colloidal silica dioxide, and sweetening agents such as saccharose or saccharin could be added or flavourings such as peppermint, methyl salicylate or orange flavouring. When the unit dosage form is a capsule it could contain in addition of the type above a liquid carrier such as polyethylene glycol or a fatty oil. Other unit dosage forms could contain other different materials that modify the physical form of the unit dosage form, e.g. as coatings. Accordingly, tablets or pills could be coated with sugar, shellac or other enteric coating agents. A syrup could in addition to the active ingredient contain saccharose as a sweetening agent and some preservatives, dyes and flavouring agents. Materials that are used for preparation of these different compositions should be pharmaceutically pure and non-toxic in the amounts used.

For parenteral administration the compounds according to the invention could be incorporated in a solution or suspension. Parenteral administration refers to the administration not through the alimentary canal but rather by injection through some other route, as subcutaneous, intramuscular, intraorbital, intracapsular, intraspinal, intrasternal, intravenous, intranasal, intrapulmonary, through the urinary tract, through the lactiferous tract in cattles, into an organ such as bone marrow, etc. Bone marrow may also be treated *in vitro.* These preparations could contain at least 0.1% by weight of an active compound according to the invention but could be varied to be approximately 0.1-50% thereof by weight. The amount of the active ingredient that is contained in such compositions is so high that a suitable dosage is obtained. The solutions or suspensions could also comprise at least one of the following adjuvants: sterile diluents such as water for injection, saline, fixed oils, polyethylene glycols, glycerol, propylene glycol or other synthetic solvents, antibacterial agents such as benzyl alcohol or methyl paraben, antioxidants such as ascorbic acid or sodium bisulfite, chelating agents such as ethylene diamine tetraacetic acid, buffers such as acetates, citrates or phosphates, and agents for adjustment of the tonicity such as sodium chloride or dextrose. The parenteral preparation could be enclosed in ampoules, disposable syringes or multiple dosage vessels made of glass or plastic. For topical administration the compounds according to the invention could be incorporated in a solution, suspension, or ointment. These preparations could contain at least 0.1 % by weight of an active compound according to the invention but could be varied to be approximately 0.1-50% thereof by weight. The amount of the active ingredient that is contained in such compositions is so high that a suitable dosage is obtained. The administration could be facilitated by applying touch, pressure, massage or heat, warms, or infrared light on the skin, which leads to enhanced skin permeability. Hirvonen et al., Nat Biotechnology 1996; 14: 1710-13 describes how to enhance the transport of a drug via the skin using the driving force of an applied electric field. Preferably, iontophoresis is effected at a slightly basic pH.

CD44-HABD or a fragment thereof can be obtained by any method of recombinant expression or chemical synthesis known in the art. It can be expressed in bacterial cells as described in example 1. It can be cloned into baculovirus vectors and expressed in insect cells. It can also be expressed in mammalian cells or in any other expression system. The affinity tag can be added to the protein product and the protein can be purified using affinity chromatography with the selected tag. The affinity tags are well known in the art and include, but are not limited to, GST-tag, His-tag, S-tag, T7-tag, V5-tag, E2-tag, c-myc-tag, HA-tag, FLAG-tag. The protein may be expressed without any tag and be purified by immunoaffinity, ion exchange or gel filtration chromatography or a combination thereof. Furthermore, CD44-HABD, fragments thereof, or its analogues can be obtained by known methods of chemical synthesis including but not limited to solid-phase peptide synthesis. CD44-HABD obtained by any of the described methods is included in the present invention.

In still another aspect, the invention relates to a method for screening for a binding partner for a molecule comprising the CD44-hyaluronic acid binding domain, as well as analogues, mutants and recombinant variants thereof, comprising the steps of:
a) providing the molecule comprising the CD44-hyaluronic acid binding domain, or fragments thereof;
b) contacting a potential binding partner to said molecule; and
c) determining the effect of said molecule on said potential binding partner.

Potential methods for screening comprise:
(I) a) Incubation of HABD, HABD analogues or mutants thereof with cells, cell lysates, cellular fractions, tissues, organisms, animals or parts of organisms or animals.
   b) Purification and detection of HABD binding partners (e.g. by affinity columns, gel electrophoreses, and any detection using antibodies or protein staining or isotopes or other means of detecting HABD or tags connected to HABD.
   c) Identification of HABD binding partners by localisation in gel electrophoresis (e.g. 2D electrophoresis), use of mass spectroscopy, sequencing, antibodies or other means of identification.
   d) Determining the effect of HABD on identified binding partners or determining the effect of other interacting agents of said potential binding partner in vitro or in vivo.
   e) Using an identified HABD-binding partner to design novel inhibitors of cell proliferation and/or angiogenesis.
(II) a) Using HABD as a bait for genetic screening of DNA, cDNA, phage, peptide, protein, cell or organism libraries or screening of synthetic peptide, protein, polysaccharide, lipid, heparan sulphate or proteoglycan libraries using HABD, analogues or mutants thereof as a bait.
   b) Detection of HABD binding partners by selection markers.
   c) Identification of HABD binding partners by sequencing, hybridisation, restriction analysis, antibodies or by step-wise elimination within a library or by other means of identification.
   d) Determining the effect of HABD on identified binding partner or determining the effect of other interacting agents of said potential binding partner in vitro or in vivo.
   e) Using an identified HABD-binding partner to design novel inhibitors of cell proliferation and/or angiogenesis.

Moreover, the screening method of the invention may also be used for determining the effect of other activators or functional blocking agents of said potential binding partners.

Furthermore, a HABD-mutant, or fragment, may be used for the screening. This may provide a more specific search for finding anti-angiogenic molecules.

In one embodiment, the potential binding partner is chosen from the group comprising: proteins, glycoproteins, proteoglycans, heparan sulphates, lipids, glycans, glycosides and saccharides.

In another embodiment, the potential binding partner is a receptor molecule, or a part of a receptor molecule or a molecule binding to a cell surface receptor molecule or a molecule located at the cell surface without being a receptor molecule.

In yet another embodiment, the potential binding partner is an extracellular molecule, being localised in the extracellular matrix, tissue sinuses, lymph- or blood vessels.

In yet another aspect the invention refers to the use of a molecule comprising the CD44-hyaluronic acid binding domain, as well as analogues, recombinant and mutated variants or fragments thereof for targeting of endothelial cells. Since the CD44-HABD-molecule of the invention has shown the capacity to bind endothelial cells, it may be used to target such cells.

In one embodiment, the molecule further comprises a moiety showing chemotherapeutic and gene therapy properties. Hereby, the CD44-HABD-molecule of the invention, in a modified variant, may be used as an anti-tumor drug towards endothelial cells. As the skilled person in the art realises, the function that is coupled to the CD44-HABD-molecule of the invention may also have other properties than anti-tumoral, such as anti-endothelial cell proliferation and/or migration, pro-apoptotic or disrupting essential functions of endothelial cells or other vascular cells. However, a moiety having anti-tumoral properties is one preferred embodiment.

By "showing chemo-therapeutic properties" is meant that the molecule having this property has the capacity to inhibit the growth and/or kill the cells it is targeted for, as measured by use of in vitro tissue culture of cells or tissues, in vitro screening of enzymatic activity, e.g. kinase, phosphatase, glycosylation, acetylation, proteolysis, linker ligation or any other enzymatic activities, proton transfer, in vitro or in vivo screening of ion pump function, and/or in vivo or in vitro screening of cell growth, apoptosis, or other means of cell death, tumor progression, metastasis, invasion, angiogenesis and/or tissue homeostasis.

The moiety showing chemotherapeutic and/or gene therapy properties may for example be chosen from different viruses for gene therapy, various chemotherapeutics, which would be known by the skilled person of the art, naked DNA coupled to habd, mutants, or fragments thereof, as well as other DNA-carriers, including but not limited to lipids, peptides and proteins.

For example, Arap et al. (Science, 1998, 279: 377-380), Ellerby et al. (Nature Medicine, 1999,5;9:1032-1038), and Trepel et al. (Human Gene Therapy, 2000, 11:1971-1981) discloses the coupling of a doxorubicin molecule (cytostatica), the coupling of an apoptosis-inducing peptide, and the coupling of a virus, respectively, for targeting of endothelial cells. These documents are hereby incorporated as a reference.

The coupling of a virus is an example on how an endothelial-targeting molecule can be used for gene therapy.

Accordingly, in yet another embodiment, the invention refers to a molecule comprising the CD44-hyaluronic acid binding domain, as well as analogues, recombinant and mutated variants or fragments thereof, and a moiety showing chemotherapeutic and/or gene therapeutical properties.

In still another embodiment, the invention refers to a molecule of the invention coupled to a moiety having chemotherapeutic properties as defined above, for medical use.

The invention will now be described with reference to the following examples, which are intended for illustrative purposes only, and do not limit the scope of the invention in any way.

### Examples

### Example 1 - Construction and purification of wild-type and mutant human CD44 HA binding domains as GST fusion proteins

Human CD44 standard isoform cDNA [Stamenkovic et al., EMBO J 1991; 10: 343-8] was used to PCR amplify the hyaluronic acid binding domain, covering amino acids 21-132, with the oligonucleotides 5'CGCGAATTCCAGATCGATTTGAATATG 3' (SEQ ID NO: 13) (containing internal EcoRl cleavage site) and 5'CGCGAGCTCCTTCTAACATGTAGTCAG 3' (SEQ ID NO: 14) (containing internal Sac1 cleavage site). The resulting PCR amplification product was cloned into a pGEX-KG vector [Guan and Dixon, Anal Biochem 1991; 192: 262-7]. Generation of CD44HABD hyaluronic acid non-binding mutant was performed by site-directed mutagenesis according to the manufacurer's protocol (Quickchange®, Stratagene). Mutagenic oligo pairs R41A
(5'GAGAAAAATGGTGCCTACAGCATCTCTCGG-3' (SEQ ID NO: 15),
5'AGATGCTGTAGGCACCATTTTTCTCCACG-3' (SEQ ID NO: 16)) and
R78SY79S (5'GACCTGCAGCTCTGGGTTCATAG 3' (SEQ ID NO: 17),
5'ATGAACCCAGAGCTGCAGGTCTC 3' (SEQ ID NO: 18)) were used for introduction of R41A and R78S, Y79S mutations respectively into wild type CD44HABD.

Chicken CAM and dog liver RNA were purified from the respective tissues using RNAqueous kit from Ambion (Austin TX) according to manufacturers specifications. CDNAs encoding chicken and dog CD44-HABD were obtained by RT-PCR with primers specific to nucleotides 63-81 and 359-330 of CD44 from the respective species. The primer pairs were as follows: 5'-CAGAGACACAATTCAATATA-3' (SEQ ID NO: 19), 5'-TTGGCTCACATGCTTTG-3' (SEQ ID NO: 20) for chicken and 5'-CGCAGATCGATTTGAACATA-3' (SEQ ID NO: 21), 5'-CCGATGTACAATCCTCTTC-3' (SEQ ID NO: 22) for dog. The cDNAs corresponding to SEQ ID NO 3 (dog) and SEQ ID NO 5 (chicken) were cloned into bacterial expression vector pET15b (Novagen) that expresses proteins in E. Coli as fusions with His-tag. Wild type and R41A, R78S, Y79S mutant GST-CD44HABD expression was induced in *E*. *coli* BL21 strain at 27°C with 1 mM IPTG at OD₆₀₀ = 0.7 and purified using glutathione agarose beads (Sigma) according to manufacturer's protocol. The resulting protein was essentially pure as detected by Coomassie Brilliant Blue staining of the preparation separated by SDS polyacrylamide electrophoresis (Figure 1.). Chicken and dog CD44HABD were purified using the HICAM Resin (Sigma) according to manufacturer's protocol. The resulting protein was also essentially pure and free of contaminants as judged by Commassie Brilliant Blue staining of SDS-polyacrylamide gels.

### Example 2 - Recombinant wild-type but not mutant CD44HABD can bind hyaluronic acid (HA) in a dose-dependent manner and can inhibit haptotaxis of human aortic endothelial cells (HAEC) towards HA.

High molecular weight hyaluronic acid at 1 mg ml⁻¹ (Sigma) in PBS was used to coat Maxisorp (Nunc) plates overnight at room temperature (RT). Wells were washed with PBS and blocked with 2% BSA for 2 h at RT. Purified proteins diluted in PBS were added to the wells and incubated 1 h at RT. After three times washing with PBS-T, mouse anti GST antibody B-14 (Santa Cruz Biotechnology) was incubated 1 h at RT before further washing and 1 h incubation at RT with HRP-conjugated goat anti mouse secondary antibody (Dako). HA binding was visualized by with OPD chromogenic substrate (Sigma) and absorbance was read at 450 nm. As shown in Figure 2A, wild type but not mutant CD44 fusion proteins bind HA in a concentration dependent manner.

Human aortic endothelial cells (HAEC) were obtained from Clonetics and grown in EBM-2 media (Clonetics) supplemented with 10% FCS, 2 µg ml⁻¹ mouse EGF (Sigma) and 50 µg ml⁻¹ gentamycin. Cell migration assay was performed in Transwell migration chambers (pore size 8mm; Costar). Lower compartment of chambers contained 1 µg ml⁻¹ high molecular weight hyaluronic acid (Sigma). CD44HABD, CD44HABD_{R41A} or GST (10 µg ml⁻¹) was added to the lower compartment. For antibody inhibition assay, cells were preincubated 30 min with 10 µg ml⁻¹ anti CD44 mAb A3 (Guo et al., 1993, 1994). Aortic endothelial cells were added to the upper compartment of the Transwell chamber and allowed to migrate to the underside of the membrane for 2.5 h. The migrated cells were fixed and stained with 0.5% crystal violet. After washing membranes were dried and bound dye was eluted with 10% acetic acid. Optical density of recovered elute was spectrophotometrically read at 600 nm. The results shown in Fig. 2B demonstrate that wild type CD44HABD but not respective non-HA-binding R41A mutant, inhibited human aortic endothelial cell migration towards HA whereas migration was also inhibited by antibody that specifically blocks CD44 binding to HA (A3).

### Example 3 - Recombinant CD44 fusion proteins block angiogenesis in chick CAM independent on HA-binding

10-day-old chick embryos were prepared as described in [Brooks et al., J Clin Invest 1995; 96: 1815-22]. For angiogenesis assay, filter discs soaked with 100 ng ml⁻¹ VEGF (Sigma), 100 ng ml⁻¹ TGFα (Sigma) or 1 µg ml⁻¹ bFGF (Gibco Lifetech) were placed on CAMs, followed by daily ectopical addition of 10 µg of CD44HABD, CD44HABD_{R41AR78SY79S} or GST and PBS as controls (n=6 per group). After 72 h, filter discs and the surrounding CAM tissue were dissected and angiogenesis quantified in a dissection microscope. Angiogenesis was assessed as the number of blood vessel branch points within the CAM area directly under the filter discs.

GST-CD44HABD and GST-CD44HABD_{R41AR78SY79S} but not GST treatment completely abolished the angiogenic effect of VEGF, bFGF or TGFα (**Fig. 3a****-d**), indicating that soluble CD44HABD blocks angiogenesis induced by three distinct angiogenic factors and. This inhibition is independent on HA binding since HA non-binding mutants were equally effective in inhibiting angiogenesis.

### Example 4 - Recombinant CD44HABD proteins block the growth of different tumour cell lines on chick CAM

SMMU-1 human melanoma cells were originally isolated from primary tumour and is CD44-negative (Guo et al., Cancer Res 1994; 54: 1561-5). HepG2 human hepatocellular carcinoma was grown in RPMI1640 containing 10% fetal bovine serum and 5 0 mgml⁻¹ ginomycin. SMMU-1-cells and M21 cells were grown in DMEM containing 10% fetal bovine serum and 50 µg ml⁻¹ gentamycin. The cells were detached from the plates by trypsinization and 1 million cells were seeded onto the CAMS of 10-day old chicken embryos. The tumours were treated every two days with 10 µg of the fusion protein of either human or chicken origin in 100 µl of PBS or with vehicle alone. 7 days later the tumours were resected and the wet mass was determined. As shown in Fig. 4 the tumour growth of all tested tumour cell lines was inhibited significantly.

### Example 5 - Recombinant CD44 fusion proteins inhibit tumour growth in nude mice

1 × 10⁶ SMMU-1 cells were injected subcutaneously into backs of 6-week old female BALB/cABom nude mice (M&B). Next day mice were injected subcutaneously proximal to the tumour with 2.4 mg kg⁻¹ of body weight of GST-CD44HABD, GST-CD44HABD_{R41AR78SY79S} or GST alone in 100 µl PBS. The treatment was repeated every second day and animals were sacrificed after two weeks, tumours dissected and analyzed for weight and prepared for tissue analysis. Subcutaneous treatment of mice with CD44HABD or non-HA-binding mutant CD44HABD _{R41AR78SY79S} significantly reduced tumour growth when compared to GST-treated controls (**Fig. 5a****, c**, P<0.05 at all time points). At day 16, when mice were sacrificed, CD44HABD and CD44HABD_{R41AR78SY79S} treated mice had in average 45% smaller tumour burden (47% and 43% respectively) than GST-treated mice (**Fig**. **5b**).

For immunohistochemical analysis, 4 µm thick tissue sections were cut from formalin fixed and paraffin embedded SMMU-1 tumours of similar size. Blood vessel staining on tissue sections was performed using goat anti-mouse PECAM-1 (Santa Cruz Biotech) primary antibody and primary antibody binding was detected by alkaline phosphatase conjugated anti-goat secondary antibodies and developed using Vectastain kit (Vector Laboratories). Immunohistochemical analysis of tumours by staining for PECAM-1 postive blood vessels showed that CD44HABD and CD44HABD_{R41AR78SY79} treated tumours were also less vascularized at the tumour border **(****Fig.5d****).**

### Example 6 - CD44-HABD fusion proteins inhibit the growth of human pancreatic cancer also in nude mice.

1 × 10⁶ BxPC-3 (ATCC, Manassas, Virginia) cells were injected subcutaneously into backs of 6-8 week old female BALB/cABom nude mice (M&B, Ry, Denmark). When tumour nodules appeared mice started to receive by subcutaneous injections proximal to the tumour 20 µg (BxPC-3) or 50 µg (SMMU-1) of GST-CD44HABD, GST-CD44HABD^{R41AR7SSY79S} or GST in 100 µl PBS. The treatment was repeated in every second day and animals were sacrificed when most of control tumours reached 25 mm in diameter. Tumour volume was calculated using formula (Width²×Length) x 0.52. At the end of experiment tumours were dissected out, analysed for weight and prepared for tissue analysis. BxPC-3 cells gave rise to slowly growing tumours. The GST-treated controls reached the average weight of 0.267 ± 0.042 g at day 52 when mice were sacrificed (Fig. 2*d-g*). The treatment of mice with GST-CD44HABD or GST-CD44HABD^{R41AR78SY79S} significantly inhibited BxPC-3 tumour growth reducing the average tumour weight by 60% (0.108 ± 0.028 g) and 70% (0.085 ± 0.017 g) compared to control, respectively (*P* < 0.05; *n* = 6).

### Example 7 - Recombinant CD44HABD inhibits specifically endothelial cell proliferation in vitro and blocks endothelial cell cycle.

For cell cycle analysis exponentially growing primary human vascular endothelial cells (HUVEC), cow pulmonary arterial endothelial (CPAE) cells, primary human fibroblasts (NHDF), MCF-7 or SMMCT1 cells were incubated 48 h in the presence of 30 µg/ml GST-CD44HABD, GST-CD44HABD^{R41AR78SY79S}, GST or PBS. Cells were pulsed with 30 µg/ml bromodeoxyuridine (BrdU) for 60 min, harvested and fixed in ice-cold ethanol. Cells were then stained for BrdU with anti-BrdU mAb G3G4 (Developmental Studies Hybridoma Bank, University of Iowa, Iowa) diluted 1:50 followed by fluorescein isothiocyanate-conjugated goat anti-mouse antibody (Jackson Immunoresearch, West Grove, Pennsylvania) in parallel with staining with propidium iodide. The cell cycle distribution was then analysed with a FACScan Flow Cytometer (Becton Dickinson, Franklin Lakes, New Jersey). Human vascular endothelial cells and cow pulmonary arterial endothelial cells exposed to GST-CD44HABD or GST-CD44HABD^{R41AR78SY79S} displayed a markedly reduced amount of cells in S-phase (5% and 6%, respectively) as compared to control treated cells (25%; Fig. 7D). Furthermore, CD44HABD had no significant effect on the cell cycle of primary human fibroblasts (NHDF) or on any of the tumour cells tested, suggesting that cell cycle inhibition by CD44HABD is specific for endothelial cells.

### Example 8 - Central domain in CD44HABD is important for its activity in inhibiting endothelial cell proliferation.

To start to analyze the regions in CD44 HABD that are important for inhibiting endothelial cell proliferation, several deletion mutants of CD44 were made using PCR. Triple mutant CD44HABD was used as a template. Oligonucleotides specific for CD44HABD were used together with a vector-specific primer for PCR amplification and the products were ligated to get vectors producing aa 21-60, 21-100 or 93-132 of CD44HABD, respectively. The sequences of wt and 3mut CD44HABD together with deletion mutants 21-100, 21-60 and 93-130 are shown in Fig. 8A, the sequences are listed as SEQ ID No. 23 (21-100, SEQ ID No. 24 (21-60) and SEQ ID No. 25 (93-132). The sequence of CD44HABD central domain involved in inhibition of endothelial cell proliferation is shown in SEQ ID No. 26. All the recombinant proteins were produced in bacteria and purified as described in Example 1.

Exponentially growing cow pulmonary endothelial cells (CPAE) were treated 48 h with indicated proteins (final conc. 15 µg/ml and 30 µg/ml, respectively) in a medium containing 10% serum. Cell proliferation was assayed by photomicrography (Fig 8B) or by MTT staining (Sigma) and spectrophotometry (Fig 8C). As shown in Fig 8C, CD44HABD aa 21-100 but not 21-60 or 93-130. Therefore, the sequences important for inhibition of endothelial cell proliferation comprises for example the sequences mapped to the region of aa 61-100 of CD44 HABD (SEQ ID No. 26). However, contribution by the flanking sequences within the entire CD44HABD is not excluded.

## Claims

1. Use of a molecule comprising a non-HA-binding variant of the CD44-hyaluronic acid binding domain (CD44-HABD), as well as non-HA-binding analogues, recombinant and mutated variants or fragments thereof, for the manufacturing of a medicament for treating states related to the inhibition of angiogenesis and/or endothelial cell proliferation, such as ocular diseases causing blindness, or impaired vision, such as macular degeneration, diabetic retinopathy and states of retinal hypoxia, states of chronical inflammation, such as rheumatoid arthritis, in psoriasis, atherosclerosis, restenosis, as well as in cancer growth and metastasis, as well as all forms of cancer diseases and tumours, such as a cancer of breast, prostate, colon, lung, skin, liver, brain, ovary, testis, skeleton, epithelium, endothelium, pancreas, kidney, muscle, adrenal gland, intestines, endocrine glands, oral cavities, head and neck, or other solid tissue origin, or being any form of leukemia, as well as in hemangioma, whereby the CD44-hyaluronic acid binding domain has a homology to the sequence SEQ ID NO:2 over the entire length of at least 55 %, more preferably at least 65 %, most preferably at least 75 %.

2. Use according to claim 1, whereby the CD44-HABD comprises at least one mutation, thereby rendering it non-HA-binding.

3. Use according to claim 2, wherein the mutation(s) is (are) chosen from F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A and F119Y, preferably R41A, R78S and Y79S.

4. Use according to claim 1-3, whereby the recombinant variant is a fusion protein having a CD44-HABD part and a GST-part, wherein the CD44-HABD-part is in a non-HA-binding form.

5. Use according to any one of the preceding claims, whereby the molecule comprising a non-HA-binding variant of the CD44-hyaluronic acid binding domain is chosen from the group comprising: human CD44-HABD (SEQ ID NO:2), dog CD44-HABD (SEQ ID NO:4), chick CD44-HABD (SEQ ID NO:6), human CD44-HABD-R41A (SEQ ID NO:8), human CD44-HABD-R78SY79S (SEQ ID NO:10), CD44-HABD-R41AR78SY79S (SEQ ID NO:12), CD44-HABD (21-100) (SEQ ID NO:23) and CD44-HABD (61-100) (SEQ ID NO:26), the sequences above further comprising at least one modification thereby making them non-HA-binding.

6. Use according to any one of the preceding claims, wherein the molecule is of human, dog, chick, primate, rat or mouse origin.

7. Use according to any one of the preceding claims, whereby the state to be treated is chosen from the following group: ocular diseases causing blindness, or impaired vision, such as macular degeneration, diabetic retinopathy and states of retinal hypoxia, states of chronical inflammation, such as rheumatoid arthritis, in psoriasis, atherosclerosis, restenosis, as well as in cancer growth and metastasis, as well as all forms of cancer diseases and tumours, such as a cancer of breast, prostate, colon, lung, skin, liver, brain, ovary, testis, skeleton, epithelium, endothelium, pancreas, kidney, muscle, adrenal gland, intestines, endocrine glands, oral cavities, head and neck, or other solid tissue origin, or being any form of leukemia, as well as in hemangioma.

8. Recombinant molecule comprising a CD44-HABD-part, and a part chosen from (i) a GST-part (ii) a moiety enabling targeting of endothelial cells for therapeutic properties, chosen from genetherapeutical or chemotherapeutical properties, and (iii) a tag chosen from IgG, IgM, IgA, His, HA, FLAG, c-myc and EGFP, wherein the CD44-HABD-part is mutated by at least one mutation that makes it non-HA-binding, such as a mutation chosen from F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A, F119Y, preferably R41A, R78S and Y79S.

9. Recombinant molecule according to claim 8, whereby the CD44-HABD-part is defined by a modified variant of any one of the amino acid sequences SEQ ID NO:2, SEQ ID NO:4, SEQ ID NO:6, SEQ ID NO:8, SEQ ID NO:10, SEQ ID NO:12, SEQ ID NO:23, SEQ ID NO:26.

10. Recombinant molecule according to claim 8 or 9, wherein the CD44-HABD part is encoded by a sequence having at least 55% homology, preferably 65% homology, more preferably 75% homology over the entire length, most preferably being any one of the nucleotide sequences: SEQ ID NO:1, SEQ ID NO:3, SEQ ID NO:5, SEQ ID NO:7, SEQ ID NO:9, SEQ ID NO:11, whereby the nucleotide sequences are in a modified form.

11. Recombinant molecule according to any one of claim 8-10, for medical use.

12. Pharmaceutical composition comprising at least one molecule according to any one of claim 8-11, in mixture or otherwise together with at least one pharmaceutically acceptable carrier or excipient.

13. Method for screening for a binding partner for a molecule according to any one of claim 8-11, comprising the steps of:
a) providing the molecule according to claim 8-11;
b) contacting a potential binding partner to said molecule; and
c) determining the effect of said molecule on said potential binding partner.

14. Method according to claim 13, whereby the potential binding partner is chosen from the group comprising: proteins, glycoproteins, proteoglycans, heparan sulphates, lipids, glycans, glycosides and saccharides.

15. Method according to claim 13 or 14, whereby the potential binding partner is a receptor molecule, a part of a receptor molecule, a molecule binding to a cell surface receptor molecule or a molecule located at the cell surface without being a receptor molecule.

## Patentansprüche

1. Verwendung eines Moleküls umfassend eine nicht-HA-bindende Variante der CD44-Hyaluronsäure-bindenden Domäne (CD44-HABD) sowie nicht-HA-bindende Analoga, rekombinante und mutierte Varianten oder Fragmente davon, für die Herstellung eines Arzneimittels zur Behandlung von Zuständen, die mit der Hemmung der Angiogenese und/oder Endothelzellproliferation in Verbindung stehen, wie Augenerkrankungen, die Blindheit oder beeinträchtigtes Sehvermögen verursachen, wie Makuladegeneration, diabetische Retinopathie und hypoxische Zustände der Netzhaut, chronische Entzündungszustände wie rheumatoide Arthritis, bei Psoriasis, Atherosklerose, Restenose, sowie bei Krebswachstum und Metastasen, sowie allen Formen von Krebserkrankungen und Tumoren, wie Krebs der Brust, Prostata, Dickdarm, Lunge, Haut, Leber, Hirn, Ovar, Testis, Skelett, Epithel, Endothel, Bauchspeicheldrüse, Niere, Muskel, Nebenniere, Eingeweide, endokrine Drüsen, Mundhöhle, Kopf und Nacken oder von sonstigen festen Gewebe abstammend oder jegliche Leukämieform, sowie bei Hämangiom, wobei die CD44-Hyaluronsäure-bindende Domäne eine Homologie über die gesamte Länge zu der Sequenz SEQ ID NO: 2 von zumindest 55%, stärker bevorzugt zumindest 65%, am meisten bevorzugt zumindest 75% hat.

2. Verwendung nach Anspruch 1, wobei die CD44-HABD zumindest eine Mutation umfasst, wodurch sie nicht-HA-bindend wird.

3. Verwendung nach Anspruch 2, wobei die Mutation(en) ausgewählt ist (sind) aus F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A und F119Y, vorzugsweise R41A, R78S und Y79S.

4. Verwendung nach den Ansprüchen 1 bis 3, wobei die rekombinante Variante ein Fusionsprotein mit einem CD44-HABD-Teil und einem GST-Teil ist, wobei der CD44-HABD-Teil in einer nicht-HA-bindenden Form ist.

5. Verwendung nach einem der vorhergehenden Ansprüche, wobei das Molekül, das eine nicht-HA-bindende Variante der CD44-Hyaluronsäure-bindenden Domäne umfasst, ausgewählt ist aus der Gruppe umfassend: menschliches CD44-HABD (SEQ ID NO: 2), Hunde-CD44-HABD (SEQ ID NO: 4), Hühner-CD44-HABD (SEQ ID NO: 6), menschliches CD44-HABD-R41A (SEQ ID NO: 8), menschliches CD44-HABD-R78SY79S (SEQ ID NO: 10), CD44-HABD-R41AR78SY79S (SEQ ID NO: 12), CD44-HABD (21-100) (SEQ ID NO: 23) und CD44-HABD (61-100) (SEQ ID NO: 26), wobei die vorstehenden Sequenzen ferner zumindest eine Modifikation umfassen, wodurch diese nicht-HA-bindend werden.

6. Verwendung nach einem der vorhergehenden Ansprüche, wobei das Molekül vom Mensch, Hund, Huhn, Primat, Ratte oder Maus stammt.

7. Verwendung nach einem der vorhergehenden Ansprüche, wobei der zu behandelnde Zustand ausgewählt ist aus der nachstehenden Gruppe: Augenerkrankungen, die Blindheit oder beeinträchtigtes Sehvermögen verursachen, wie Makulardegeneration, diabetische Retinopathie und hypoxische Zustände der Netzhaut, chronische Entzündungszustände wie rheumatoide Arthritis, bei Psoriasis, Atherosklerose, Restenose, sowie bei Krebswachstum und Metastasen, sowie allen Formen von Krebserkrankungen und Tumoren, wie Krebs der Brust, Prostata, Dickdarm, Lunge, Haut, Leber, Hirn, Ovar, Testis, Skelett, Epithel, Endothel, Bauchspeicheldrüse, Niere, Muskel, Nebenniere, Eingeweide, endokrine Drüsen, Mundhöhle, Kopf und Nacken oder von sonstigen festen Gewebe abstammend oder jegliche Leukämieform, sowie bei Hämangiom.

8. Rekombinantes Molekül umfassend einen CD44-HABD-Teil und einen Teil ausgewählt aus (i) einem GST-Teil, (ii) einem Teil, das Targeting von Endothelzellen für therapeutische Eigenschaften ermöglicht, ausgewählt aus gentherapeutischen oder chemotherapeutischen Eigenschaften, und (iii) einer Markierung ausgewählt aus IgG, IgM, IgA, His, HA, FLAG, c-myc und EGFP, wobei der CD44-HABD-Teil mit zumindest einer Mutation mutiert ist, wodurch er nicht-HA-bindend gemacht wird, wie eine Mutation ausgewählt aus F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A, F119Y, vorzugsweise R41A, R78S und Y79S.

9. Rekombinantes Molekül nach Anspruch 8, wobei der CD44-HABD-Teil definiert ist durch eine modifizierte Variante einer der Aminosäuresequenzen SEQ ID NO: 2, SEQ ID NO: 4, SEQ ID NO: 6, SEQ ID NO: 8, SEQ ID NO: 10, SEQ ID NO: 12, SEQ ID NO: 23, SEQ ID NO: 26.

10. Rekombinantes Molekül nach Anspruch 8 oder 9, wobei der CD44-HABD-Teil durch eine Sequenz mit zumindest 55% Homologie, bevorzugt 65% Homologie, stärker bevorzugt 75% Homologie über die gesamte Länge codiert wird, am meisten bevorzugt eine der Nucleotidsequenzen: SEQ ID NO: 1, SEQ ID NO: 3, SEQ ID NO: 5, SEQ ID NO: 7, SEQ ID NO: 9, SEQ ID NO: 11 ist, wobei die Nucleotidsequenzen in einer modifizierten Form sind.

11. Rekombinantes Molekül nach einem der Ansprüche 8 bis 10 zur medizinischen Verwendung.

12. Arzneimittel umfassend zumindest ein Molekül nach einem der Ansprüche 8 bis 11, im Gemisch oder in einer anderen Weise zusammen mit zumindest einem pharmazeutisch verträglichen Träger oder Exzipienten.

13. Screeningverfahren für einen Bindungspartner eines Moleküls nach einem der Ansprüche 8 bis 11, umfassend die Schritte:
(a) Bereitstellen des Moleküls nach Anspruch 8 bis 11;
(b) Kontaktieren eines potentiellen Bindungspartners mit dem Molekül; und
(c) Bestimmen des Effekts des Moleküls auf den potentiellen Bindungspartner.

14. Verfahren nach Anspruch 13, wobei der potentielle Bindungspartner ausgewählt ist aus der Gruppe umfassend: Proteine, Glycoproteine, Proteoglycane, Heparansulfate, Lipide, Glycane, Glycoside und Saccharide.

15. Verfahren nach Anspruch 13 oder 14 wobei der potentielle Bindungspartner ein Rezeptormolekül, ein Teil eines Rezeptormoleküls, ein Molekül, das an ein Zelloberflächenrezeptormolekül bindet, oder ein Molekül ist, das sich auf der Oberfläche einer Zelle befindet, ohne dass es ein Rezeptormolekül ist.

## Revendications

1. Utilisation d'une molécule comprenant un variant ne liant pas HA du domaine de liaison de l'acide hyaluronique de CD44 (CD44-HABD), ainsi que des analogues, des variants recombinants et mutés ne liants pas HA ou des fragments de ceux-ci, pour la fabrication d'un médicament pour traiter les états liés à l'inhibition de l'angiogenèse et/ou la prolifération des cellules endothéliales, notamment les maladies oculaires provoquant la cécité, ou une vision altérée, notamment une dégénérescence maculaire, une rétinopathie diabétique et les états d'hypoxie rétinienne, les états d'inflammation chronique, notamment la polyarthrite rhumatoïde, dans le psoriasis, l'athérosclérose, la resténose, ainsi que dans la croissance et la métastase du cancer, ainsi que toutes les formes de maladies et tumeurs cancéreuses, notamment le cancer du sein, de la prostate, du côlon, du poumon, de la peau, du foie, du cerveau, de l'ovaire, du testicule, du squelette, de l'épithélium, de l'endothélium, du pancréas, du rein, du muscle, de la glande surrénale, des intestins, des glandes endocrines, des cavités buccales, de la tête et du cou, ou d'une autre origine du tissu solide, ou une quelconque forme de leucémie, ainsi que dans l'hémangiome, moyennant quoi le domaine de fixation de l'acide hyaluronique de CD44 a une homologie avec la séquence SEQ ID n° : 2 sur toute la longueur d'au moins 55 %, plus préférablement d'au moins 65 %, le plus préférablement d'au moins 75 %.

2. Utilisation selon la revendication 1, moyennant quoi le CD44-HABD comprend au moins une mutation, le rendant ainsi non liant à HA.

3. Utilisation selon la revendication 2, dans laquelle la ou les mutation(s) est/sont choisie(s) parmi F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A et F119Y, de préférence R41A, R78S et Y79S.

4. Utilisation selon la revendication 1-3, moyennant quoi le variant recombinant est une protéine de fusion ayant une partie CD44-HABD et une partie GST, dans laquelle la partie CD44-HABD est sous une forme ne liant pas HA.

5. Utilisation selon l'une quelconque des revendications précédentes, moyennant quoi la molécule comprenant un variant ne liant pas HA du domaine de liaison de l'acide hyaluronique de CD44 est choisie dans le groupe comprenant : CD44-HABD d'humain (SEQ ID n° : 2), CD44-HABD de chien (SEQ ID n° : 4), CD44-HABD de poulet (SEQ ID n° : 6), CD44-HABD-R41A d'humain (SEQ ID n° : 8), CD44-HABD-R78SY79S d'humain (SEQ ID n° : 10), CD44-HABD-R41AR78SY79S (SEQ ID n° : 12), CD44-HABD (21-100) (SEQ ID n° : 23) et CD44-HABD (61-100) (SEQ ID n° : 26), les séquences ci-dessus comprenant en outre au moins une modification les rendant ainsi non liantes à HA.

6. Utilisation selon l'une quelconque des revendications précédentes, dans laquelle la molécule provient de l'humain, du chien, du poulet, d'un primate, du rat ou de la souris.

7. Utilisation selon l'une quelconque des revendications précédentes, moyennant quoi l'état à traiter est choisi dans le groupe suivant : les maladies oculaires provoquant la cécité, ou une vision altérée, notamment une dégénérescence maculaire, une rétinopathie diabétique et les états d'hypoxie rétinienne, les états d'inflammation chronique, notamment la polyarthrite rhumatoïde, dans le psoriasis, l'athérosclérose, la resténose, ainsi que dans la croissance et la métastase du cancer, ainsi que toutes les formes de maladies et tumeurs cancéreuses, notamment le cancer du sein, de la prostate, du côlon, du poumon, de la peau, du foie, du cerveau, de l'ovaire, du testicule, du squelette, de l'épithélium, de l'endothélium, du pancréas, du rein, du muscle, de la glande surrénale, des intestins, des glandes endocrines, des cavités buccales, de la tête et du cou, ou d'une autre origine du tissu solide, ou une quelconque forme de leucémie, ainsi que dans l'hémangiome.

8. Molécule recombinante comprenant une partie CD44-HABD, et une partie choisie parmi (i) une partie GST, (ii) un fragment permettant le ciblage des cellules endothéliales pour les propriétés thérapeutiques, choisies parmi les propriétés de thérapie génique ou chimiothérapeutiques, et (iii) une étiquette choisie parmi IgG, IgM, IgA, His, HA, FLAG, c-myc et EGFP, dans laquelle la partie CD44-HABD est mutée par au moins une mutation qui la rend non liante à HA, notamment une mutation choisie parmi F34A, F34Y, K38R, K38S, R41A, Y42F, Y42S, R46S, E48S, K54S, Q65S, K68S, R78K, R78S, Y79F, Y79S, N100A, N100R, N101S, Y105F, Y105S, S112R, Y114F, F119A et F119Y, de préférence R41A, R78S et Y79S.

9. Molécule recombinante selon la revendication 8, moyennant quoi la partie CD44-HABD est définie par un variant modifié de l'une quelconque des séquences d'acides aminés SEQ ID n° : 2, SEQ ID n° : 4, SEQ ID n° : 6, SEQ ID n° : 8, SEQ ID n° : 10, SEQ ID n° : 12, SEQ ID n° : 23, SEQ ID n° : 26.

10. Molécule recombinante selon la revendication 8 ou 9, moyennant quoi la partie CD44-HABD est codée par une séquence ayant au moins 55 % d'homologie, de préférence 65 % d'homologie, plus préférablement 75 % d'homologie sur toute la longueur, le plus préférablement étant l'une quelconque des séquences nucléotidiques : SEQ ID n° : 1, SEQ ID n° : 3, SEQ ID n° : 5, SEQ ID n° : 7, SEQ ID n° : 9, SEQ ID n° : 11, moyennant quoi les séquences nucléotidiques sont sous une forme modifiée.

11. Molécule recombinante selon l'une quelconque des revendications 8 à 10, pour un usage médical.

12. Composition pharmaceutique comprenant au moins une molécule selon l'une quelconque des revendications 8 à 11, dans un mélange ou avec au moins un support ou excipient pharmaceutiquement acceptable.

13. Procédé de criblage d'un partenaire de liaison pour une molécule selon l'une quelconque des revendications 8 à 11, comprenant les étapes consistant à :
a) fournir la molécule selon les revendications 8 à 11 ;
b) mettre en contact un partenaire de liaison potentiel avec ladite molécule ; et
c) déterminer l'effet de ladite molécule sur ledit partenaire de liaison potentiel.

14. Procédé selon la revendication 13, moyennant quoi le partenaire de liaison potentiel est choisi dans le groupe comprenant : les protéines, les glycoprotéines, les sulfates d'héparane, les lipides, les glycanes, les glycosides et les saccharides.

15. Procédé selon la revendication 13 ou 14, moyennant quoi le partenaire de liaison potentiel est une molécule réceptrice, une partie d'une molécule réceptrice, une molécule se liant à une molécule de récepteur de surface cellulaire ou une molécule située au niveau de la surface cellulaire sans être une molécule réceptrice.


### SEQUENCE LISTING

<110> Strömblad, Staffan
   Pall, Taavi
   Kogerman, Priit
<120> new drug
<130> cd44habd
<150> SE0102823-2
   <151> 2001-08-24
<150> US 60/314,971
   <151> 2001-08-24
<160> 26
<170> PatentIn version 3.1
<210> 1
   <211> 336
   <212> DNA
   <213> homo sapiens
<400> 1
   cagatcgatt tgaatataac ctgccgcttt gcaggtgtat tccacgtgga gaaaaatggt     60
   cgctacagca tctctcggac ggaggccgct gacctctgca aggctttcaa tagcaccttg    120
   cccacaatgg cccagatgga gaaagctctg agcatcggat ttgagacctg caggtatggg    180
   ttcatagaag ggcatgtggt gattccccgg atccacccca actccatctg tgcagcaaac    240
   aacacagggg tgtacatcct cacatacaac acctcccagt atgacacata ttgcttcaat    300
   gcttcagctc cacctgaaga agattgtaca tcagtc                              336
<210> 2
   <211> 112
   <212> PRT
   <213> homo sapiens
<400> 2
   Gln Ile Asp Leu Asn Met Thr Cys Arg Phe Ala Gly Val Phe His Val
   1               5                   10                  15
   Glu Lys Asn Gly Arg Tyr Ser Ile Ser Arg Thr Glu Ala Ala Asp Leu
               20                  25                  30
   Cys Lys Ala Phe Asn Ser Thr Leu Pro Thr Met Ala Gln Met Glu Lys
           35                  40                  45
   Ala Leu Ser Ile Gly Phe Glu Thr Cys Arg Tyr Gly Phe Ile Glu Gly
       50                  55                  60
   His Val Val Ile Pro Arg Ile His Pro Asn Ser Ile Cys Ala Ala Asn
   65                  70                  75                  80
   Asn Thr Gly Val Tyr Ile Leu Thr Ser Asn Thr Ser Gln Tyr Asp Thr
                   85                  90                  95
   Tyr Cys Phe Asn Ala Ser Ala Pro Pro Glu Glu Asp Cys Thr Ser Val
               100                 105                 110
<210> 3
   <211> 336
   <212> DNA
   <213> canis familiaris
<400> 3
   cgcagatcga tttgaacata acctgccgct acgcaggtgt gttccatgtg gagaaaaacg      60
   gtcgctacag catctccagg acggcggctg ccgacctctg caaggctttc aacagcaccc     120
   tgcccaccat ggcccagatg gagcgagccc tgagcgtggg ctttgagacc tgcaggtacg     180
   ggttcataga aggacatgtg gtgatccccc gtatccaacc caatgctatt tgtgctgcaa     240
   accatacagg ggtgtacatc ctcatatcca acacctccca gtacgacacg tattgcttca     300
   atgcttcagc tccacctgaa gaggattgta catcgg                               336
<210> 4
   <211> 112
   <212> PRT
   <213> canis familiaris
<400> 4
   Gln Ile Asp Leu Asn Ile Thr Cys Arg Tyr Ala Gly Val Phe His Val
   1               5                   10                  15
   Glu Lys Asn Gly Arg Tyr Ser Ile Ser Arg Thr Ala Ala Ala Asp Leu
               20                  25                  30
   Cys Lys Ala Phe Asn Ser Thr Leu Pro Thr Met Ala Gln Met Glu Arg
           35                  40                  45
   Ala Leu Ser Val Gly Phe Glu Thr Cys Arg Tyr Gly Phe Ile Glu Gly
       50                  55                  60
   His Val Val Ile Pro Arg Ile Gln Pro Asn Ala Ile Cys Ala Ala Asn
   65                  70                  75                  80
   His Thr Gly Val Tyr Ile Leu Ile Ser Asn Thr Ser Gln Tyr Asp Thr
                   85                  90                  95
   Tyr Cys Phe Asn Ala Ser Ala Pro Pro Glu Glu Asp Cys Thr Ser Val
               100                 105                 110
<210> 5
   <211> 339
   <212> DNA
   <213> gallus gallus
<400> 5
   cagagacaca attcaatata acttgcagat atggaggagt gtttcatgtg gagaaaaatg     60
   gtcgctacag tctcacacga gctgaagcaa ttgagctctg tagagctctc aatagtacct    120
   tggcaacact ggagcaattt gaaagagctc atgcacttgg atttgaaacg tgcaggtatg    180
   gttttatagt ggggcatatt gttatcccac gaatcaatcc atatcatctt tgtgcagcaa    240
   atcatacagg catttacaaa ctttcagcaa atacaactgg ccggtatgat gcatattgtt    300
   acaatgcaac agaaacgagg agcaaagcat gtgagccaa                           339
<210> 6
   <211> 113
   <212> PRT
   <213> gallus gallus
<400> 6
   Glu Thr Gln Phe Asn Ile Thr Cys Arg Tyr Gly Gly Val Phe His Val
   1               5                   10                  15
   Glu Lys Asn Gly Arg Tyr Ser Leu Thr Arg Ala Glu Ala Ile Glu Leu
               20                  25                  30
   Cys Arg Ala Leu Asn Ser Thr Leu Ala Thr Leu Val Gln Phe Glu Arg
           35                  40                  45
   Ala His Ala Leu Gly Phe Glu Thr Cys Arg Tyr Gly Phe Ile Val Gly
       50                  55                  60
   His Ile Val Ile Pro Arg Ile Asn Pro Tyr His Leu Cys Ala Ala Asn
   65                  70                  75                  80
   His Thr Gly Ile Tyr Lys Leu Ser Ala Asn Thr Thr Gly Arg Tyr Asp
                   85                  90                  95
   Ala Tyr Cys Tyr Asn Ala Thr Glu Thr Arg Ser Lys Ala Cys Glu Pro
               100                 105                 110
   Ile
<210> 7
   <211> 336
   <212> DNA
   <213> homo sapiens
<400> 7
   cagatcgatt tgaatataac ctgccgcttt gcaggtgtat tccacgtgga gaaaaatggt      60
   gcctacagca tctctcggac ggaggccgct gacctctgca aggctttcaa tagcaccttg     120
   cccacaatgg cccagatgga gaaagctctg agcatcggat ttgagacctg caggtatggg     180
   ttcatagaag ggcatgtggt gattccccgg atccacccca actccatctg tgcagcaaac     240
   aacacagggg tgtacatcct cacatacaac acctcccagt atgacacata ttgcttcaat     300
   gcttcagctc cacctgaaga agattgtaca tcagtc                               336
<210> 8
   <211> 112
   <212> PRT
   <213> homo sapiens
<400> 8
   Gln Ile Asp Leu Asn Met Thr Cys Arg Phe Ala Gly Val Phe His Val
   1               5                   10                  15
   Glu Lys Asn Gly Ala Tyr Ser Ile Ser Arg Thr Glu Ala Ala Asp Leu
               20                  25                  30
   Cys Lys Ala Phe Asn Ser Thr Leu Pro Thr Met Ala Gln Met Glu Lys
           35                  40                  45
   Ala Leu Ser Ile Gly Phe Glu Thr Cys Arg Tyr Gly Phe Ile Glu Gly
       50                  55                  60
   His Val Val Ile Pro Arg Ile His Pro Asn Ser Ile Cys Ala Ala Asn
   65                  70                  75                  80
   Asn Thr Gly Val Tyr Ile Leu Thr Ser Asn Thr Ser Gln Tyr Asp Thr
                   85                  90                  95
   Tyr Cys Phe Asn Ala Ser Ala Pro Pro Glu Glu Asp Cys Thr Ser Val
               100                 105                 110
<210> 9
   <211> 336
   <212> DNA
   <213> homo sapiens
<400> 9
   cagatcgatt tgaatataac ctgccgcttt gcaggtgtat tccacgtgga gaaaaatggt      60
   cgctacagca tctctcggac ggaggccgct gacctctgca aggctttcaa tagcaccttg     120
   cccacaatgg cccagatgga gaaagctctg agcatcggat ttgagacctg cagctctggg     180
   ttcatagaag ggcatgtggt gattccccgg atccacccca actccatctg tgcagcaaac     240
   aacacagggg tgtacatcct cacatacaac acctcccagt atgacacata ttgcttcaat     300
   gcttcagctc cacctgaaga agattgtaca tcagtc                               336
<210> 10
   <211> 112
   <212> PRT
   <213> homo sapiens
<400> 10
   Gln Ile Asp Leu Asn Met Thr Cys Arg Phe Ala Gly Val Phe His Val
   1               5                   10                  15
   Glu Lys Asn Gly Arg Tyr Ser Ile Ser Arg Thr Glu Ala Ala Asp Leu
               20                  25                  30
   Cys Lys Ala Phe Asn Ser Thr Leu Pro Thr Met Ala Gln Met Glu Lys
           35                  40                  45
   Ala Leu Ser Ile Gly Phe Glu Thr Cys Ser Ser Gly Phe Ile Glu Gly
       50                  55                  60
   His Val Val Ile Pro Arg Ile His Pro Asn Ser Ile Cys Ala Ala Asn
   65                  70                  75                  80
   Asn Thr Gly Val Tyr Ile Leu Thr Ser Asn Thr Ser Gln Tyr Asp Thr
                   85                  90                  95
   Tyr Cys Phe Asn Ala Ser Ala Pro Pro Glu Glu Asp Cys Thr Ser Val
               100                 105                 110
<210> 11
   <211> 336
   <212> DNA
   <213> homo sapiens
<400> 11
   cagatcgatt tgaatataac ctgccgcttt gcaggtgtat tccacgtgga gaaaaatggt      60
   gcctacagca tctctcggac ggaggccgct gacctctgca aggctttcaa tagcaccttg     120
   cccacaatgg cccagatgga gaaagctctg agcatcggat ttgagacctg cagctctggg     180
   ttcatagaag ggcatgtggt gattccccgg atccacccca actccatctg tgcagcaaac     240
   aacacagggg tgtacatcct cacatacaac acctcccagt atgacacata ttgcttcaat     300
   gcttcagctc cacctgaaga agattgtaca tcagtc                               336
<210> 12
   <211> 112
   <212> PRT
   <213> homo sapiens
<400> 12
   Gln Ile Asp Leu Asn Met Thr Cys Arg Phe Ala Gly Val Phe His Val
   1               5                   10                  15
   Glu Lys Asn Gly Ala Tyr Ser Ile Ser Arg Thr Glu Ala Ala Asp Leu
               20                  25                  30
   Cys Lys Ala Phe Asn Ser Thr Leu Pro Thr Met Ala Gln Met Glu Lys
           35                  40                  45
   Ala Leu Ser Ile Gly Phe Glu Thr Cys Ser Ser Gly Phe Ile Glu Gly
       50                  55                  60
   His Val Val Ile Pro Arg Ile His Pro Asn Ser Ile Cys Ala Ala Asn
   65                  70                  75                  80
   Asn Thr Gly Val Tyr Ile Leu Thr Ser Asn Thr Ser Gln Tyr Asp Thr
                   85                  90                  95
   Tyr Cys Phe Asn Ala Ser Ala Pro Pro Glu Glu Asp Cys Thr Ser Val
               100                 105                 110
<210> 13
   <211> 27
   <212> DNA
   <213> artificial
<220>
   <223> PCR primer
<400> 13
   cgcgaattcc agatcgattt gaatatg 27
<210> 14
   <211> 27
   <212> DNA
   <213> artificial
<220>
   <223> PCR primer
<400> 14
   cgcgagctcc ttctaacatg tagtcag 27
<210> 15
   <211> 30
   <212> DNA
   <213> artificial
<220>
   <223> mutation primer
<400> 15
   gagaaaaatg gtgcctacag catctctcgg 30
<210> 16
   <211> 29
   <212> DNA
   <213> artificial
<220>
   <223> mutation primer
<400> 16
   agatgctgta ggcaccattt ttctccacg 29
<210> 17
   <211> 23
   <212> DNA
   <213> artificial
<220>
   <223> mutation primer
<400> 17
   gacctgcagc tctgggttca tag 23
<210> 18
   <211> 23
   <212> DNA
   <213> artificial
<220>
   <223> mutation primer
<400> 18
   atgaacccag agctgcaggt ctc 23
<210> 19
   <211> 20
   <212> DNA
   <213> artificial
<220>
   <223> RT-PCR-primer
<400> 19
   cagagacaca attcaatata 20
<210> 20
   <211> 17
   <212> DNA
   <213> artificial
<220>
   <223> RT-PCR-primer
<400> 20
   ttggctcaca tgctttg 17
<210> 21
   <211> 20
   <212> DNA
   <213> artificial
<220>
   <223> RT-PCR-primer
<400> 21
   cgcagatcga tttgaacata 20
<210> 22
   <211> 19
   <212> DNA
   <213> artificial
<220>
   <223> RT-PCR-primer
<400> 22
   ccgatgtaca atcctcttc 19
<210> 23
   <211> 80
   <212> PRT
   <213> artificial
<220>
   <223> deletion mutant
<400> 23
   Gln Ile Asp Leu Asn Ile Thr Cys Arg Phe Ala Gly Val Phe His Val
   1               5                   10                  15
   Glu Lys Asn Gly Arg Tyr Ser Ile Ser Arg Thr Glu Ala Ala Asp Leu
               20                  25                  30
   Cys Lys Ala Phe Asn Ser Thr Leu Pro Thr Met Ala Gln Met Glu Lys
           35                  40                  45
   Ala Leu Ser Ile Gly Phe Glu Thr Cys Ser Ser Gly Phe Ile Glu Gly
       50                  55                  60
   His Val Val Ile Pro Arg Ile His Pro Asn Ser Ile Cys Ala Ala Asn
   65                  70                  75                  80
<210> 24
   <211> 40
   <212> PRT
   <213> artificial
<220>
   <223> deletion mutant
<400> 24
   Gln Ile Asp Leu Asn Ile Thr Cys Arg Phe Ala Gly Val Phe His Val
   1               5                   10                  15
   Glu Lys Asn Gly Ala Tyr Ser Ile Ser Arg Thr Glu Ala Ala Asp Leu
               20                  25                  30
   Cys Lys Ala Phe Asn Ser Thr Leu
           35                  40
<210> 25
   <211> 38
   <212> PRT
   <213> artificial
<220>
   <223> deletion mutant
<400> 25
   Ser Ile Cys Ala Ala Asn Asn Thr Gly Val Tyr Ile Leu Thr Tyr Asn
   1               5                   10                  15
   Thr Ser Gln Tyr Asp Thr Tyr Cys Phe Asn Ala Ser Ala Pro Pro Glu
               20                  25                  30
   Glu Asp Cys Thr Ser Val
           35
<210> 26
   <211> 40
   <212> PRT
   <213> artificial
<220>
   <223> deletion mutant
<400> 26
   Pro Thr Met Ala Gln Met Glu Lys Ala Leu Ser Ile Gly Phe Glu Thr
   1               5                   10                  15
   Cys Ser Ser Gly Phe Ile Glu Gly His Val Val Ile Pro Arg Ile His
               20                  25                  30
   Pro Asn Ser Ile Cys Ala Ala Asn
           35                  40